# EUROPEAN PATENT SPECIFICATION

(11) **EP 4 440 635 B1**
(45) Date of publication and mention of the grant of the patent: **11.03.2026**
(21) Application number: 22821690.9
(22) Date of filing: 01.12.2022
(51) Int. Cl.: A61L 27/24, A61L 27/48, A61L 27/54

(54) **METHOD OF PROVIDING AN IMPLANTABLE HYDROGEL MATERIAL AND AN IMPLANTABLE HYDROGEL MATERIAL**
VERFAHREN ZUR BEREITSTELLUNG EINES IMPLANTIERBAREN HYDROGELMATERIALS UND IMPLANTIERBARES HYDROGELMATERIAL
PROCÉDÉ DE FOURNITURE D'UN MATÉRIAU D'HYDROGEL IMPLANTABLE ET MATÉRIAU D'HYDROGEL IMPLANTABLE

(30) Priority: 03.12.2021 SE 2151478
(43) Date of publication of application: 09.10.2024
(73) Proprietor: Rafat, Mehrdad, 582 76 Linköping (SE)
(72) Inventor: Rafat, Mehrdad, 582 76 Linköping (SE)
(74) Representative: Zacco Sweden AB
(86) International application number: PCT/SE2022/051130
(87) International publication number: WO 2023/101594

(56) References cited:
- WO-A2-2016/178586
- US-A1- 2012 321 585
- US-A1- 2021 138 113
- TRACHE DJALAL ET AL: "Nanocellulose: From Fundamentals to Advanced Applications", FRONTIERS IN CHEMISTRY, vol. 8, no. Article : 392, 1 May 2020 (2020-05-01), pages 1 - 33, XP093021463, Retrieved from the Internet <URL:http://dx.doi.org/10.3389/fchem.2020.00392> DOI: 10.3389/fchem.2020.00392
- LU TIANHONG ET AL: "Composite aerogels based on dialdehyde nanocellulose and collagen for potential applications as wound dressing and tissue engineering scaffold", COMPOSITES SCIENCE AND TECHNOLOGY, ELSEVIER, AMSTERDAM, NL, vol. 94, 4 February 2014 (2014-02-04), pages 132 - 138, XP028632518, ISSN: 0266-3538, DOI: 10.1016/J.COMPSCITECH.2014.01.020

## Description

### TECHNICAL FIELD

The present disclosure relates to a method of providing an implantable hydrogel material, an implantable hydrogel material and use thereof.

### BACKGROUND ART

Injured and diseased tissues or organs have been traditionally treated or replaced by autologous grafts, allogenic grafts, or synthetic or natural-based biomaterials. However, there is a huge global shortage of tissue grafts. Donor tissue grafts may also cause donor site morbidity and loss of organ functionality and allogenic grafts are associated with the risk of disease transmission and often require the use of immunosuppressant drugs.

As for synthetic biomaterials, although many of them have achieved widespread clinical use, seamless integration and immunological response issues remain. These issues have led to the more recent paradigm shift to the development of tissue-engineered biomaterials that mimic the extracellular matrix (ECM) of the natural tissue.

For these biomaterials to be successful, they need to be mechanically robust and elastic to support and maintain tissue structure, and cell friendly and bio-interactive to allow seamless host-biomaterial integration that helps restore tissue functionality and avoid rejection.

Collagen is a family of ECM macromolecules within the body that contribute to both mechanical properties and biological function of various types of tissues such as cornea, skin, bone, tendons, ligaments, blood vessels, and the heart. Although native collagen is very robust and elastic due to the presence of natural crosslinks and proteoglycans, extracted collagen is rapidly degraded and lacks the mechanical toughness and elasticity, due to the dissociation of natural cross-links and removal of proteoglycans during isolation and purification process.

Chemical crosslinking techniques are often used to enhance the mechanical properties of collagen hydrogel biomaterials. This, however, may result in collagen-based scaffolds that are either too soft or too brittle and that do not actively interact with the body cells and tissues.

To improve the quality of collagen hydrogels, other materials may be incorporated in the collagen material. Lu et al., Composite aerogels based on dialdehyde nanocellulose and collagen for potential applications as wound dressing and tissue engineering scaffold, Composites Science and Technology 94 (2014) 132-138, reported preparation and characterization of a non-transparent aerogel based on collagen and aldehyde activated nanocellulose fibers (NCFs). Harsh chemicals such as sodium periodate were used to functionalize NCF aldehyde groups for crosslinking with collagen. Lohrasbi et al., Collagen/cellulose nanofiber hydrogel scaffold: physical, mechanical and cell biocompatibility properties, Cellulose 27(11), January 2020, discuss preparation and characterization of a non-transparent composite hydrogel based on collagen and cellulose nanofibers, wherein the mixture was partially stabilized by increasing pH and temperature, thereby forming a physically stabilised hydrogel. In US8691974 is shown three-dimensional bioprinting of biosynthetic cellulose implants and scaffolds for tissue engineering. Fermentation techniques are used for making biosynthetic cellulose (BC) using bacterial fermentation process controlling the 3D shape. In these implants there is no collagen.

Even though cellulose is a natural biopolymer, it is not one of the components of the ECM. Therefore, implantable scaffolds that are mainly comprised of cellulose and its derivatives alone may not be the best options for implantable devices or products.

Scaffolds made from high ratios of nanocellulose may not be permeable to nutrients and oxygen, why not ideal for tissue engineering applications at high ratios. Furthermore, the nanocellulose form (i.e., crystallinity, hydration and swelling) of cellulose may affect the degree of degradation, absorption and immune response (Lin et al., Nanocellulose in biomedicine: Current status and future prospects, European Polymer Journal 59 (2014) 302-325).

Patent application US 2012/321585 discloses a corneal implant composition, comprising collagen, the document explained how the combination of CMC (a carbodiimide) and riboflavin improves the physical properties of the gel.

In view of the above, there is a need for implantable collagen-based materials that are mechanical robust, while not disrupting the collagen backbone and therefore not compromising biological properties for implantation. The collagen-based material should also be elastic and biocompatible to support and maintain tissue structure, and further cell friendly and bio-interactive, allowing seamless host-biomaterial integration that helps restore tissue functionality and avoid rejection.

### SUMMARY OF THE INVENTION

It is an object of the present disclosure to provide an implantable collagen-based hydrogel material that alleviates or overcomes at least some of the disadvantages with known collagen-based hydrogel materials.

The invention is defined by the appended independent patent claims. Non-limiting embodiments emerge from the dependent claims, the appended drawings and the following description.

According to a first aspect, there is provided a method of providing an implantable hydrogel material as defined in the claims. The method comprises to provide a solution having a collagen concentration of 0.1 to 30 wt.% and a cellulose nanofiber concentration of 0.1 to 30 wt.%. To the solution is added a non-polymeric short-range carbodiimide crosslinking agent to a concentration of 1-30 wt.% and riboflavin to a concentration of 0.1 to 10 wt.%. Thereafter the formed intermediate hydrogel material is exposed to ultraviolet A light, thereby forming an implantable hydrogel material.

In the above-described method, an implantable collagen-based hydrogel material is formed, which comprises collagen and which is nano-reinforced with an amount of cellulose nanofibers.

With "hydrogel material" is here meant a collagen network, which exhibits the ability to swell in water or aqueous solution without dissolution, retaining a significant portion of water or aqueous solution within its structure.

The use of non-polymeric short-range carbodiimide crosslinking agent and riboflavin (vitamin B2) with ultraviolet A light (UVA) exposure results in a double chemical-photochemical crosslinking of the material, i.e., both a chemical and physical crosslinking. The double-crosslinking stabilizes collagen in a viscous form. The crosslinking agents do not become integrated within the final implantable hydrogel material, resulting in an entirely natural, chemical-free, transparent hydrogel material, stabilized collagen.

A collagen molecule cross-links with itself, also called intra-molecular cross-links, which is generally done via the short-range small carbodiimide crosslinking agent. Riboflavin is not expected to result in such crosslinks due to their larger molecular size. Riboflavin has bulky cyclic groups (e.g., isoalloxazine) that may limit their penetration depth into the molecules.

A collagen molecule cross-links with another collagen molecule in its vicinity, also called inter-molecular cross-link. This is generally done via short-range small carbodiimide crosslinkers and larger size crosslinkers such as riboflavin in the presence of UVA. The larger spacing between collagen molecules allows riboflavin to penetrate in and creates crosslinks. A collagen fiber cross-links with another collagen fiber in its vicinity, also called inter-fibrillar crosslink. Short-range small carbodiimide crosslinkers cannot produce such crosslinks because of the large spacing between the collagen fibers. However, riboflavin in the presence of UVA can result in crosslinks between two collagen fibers.

Such double-crosslinked collagen material is mechanically robust, tough, and stress tolerant, translating into an ability to withstand surgical implantation, mechanical forces and enzymatic degradation in vivo. The formed hydrogel material is not only robust, elastic, and suturable but also interact with cells and the surrounding tissues when implanted into animal and human models.

The hydrogel material is nano-reinforced with an amount of cellulose nanofibers (CNF). CNF is added to the collagen to reinforce the collagen material even more than what may be obtained by the double cross-linking of pure collagen. The CNF added to the material enhances collagen mechanical properties without changing biocompatibility, transparency and other biological properties of the implantable hydrogel material. Even though cellulose is a natural biopolymer, it is not one of the components of the extracellular matrix (ECM). Therefore, implantable scaffolds that are mainly comprised of cellulose and its derivatives alone may not be the best options for implantable devices or products.

The CNFs can be derived from various sources such as Ciona Intestinals, wood pulp, and bacterial sources. A CNF molecule cross-links with itself through intra-molecular crosslinking. This is generally done via short-range small molecule carbodiimide crosslinkers and not the larger riboflavin.

A CNF molecule cross-links with another CNF molecule, also called inter-molecular or inter-fibrillary crosslinking. This is generally done via short-range small molecule carbodiimide crosslinkers when the CNFs are in each other vicinity and/or by larger size crosslinkers such as riboflavin in the presence of UVA when CNFs are more distanced. The larger spacing between CNF molecules allows riboflavin to penetrate in and creates crosslinks. This type of crosslinks can also occur between two CNF fibers that are separately and covalently attached to two different collagen fibers creating a bridge between two collagen fibers. This is one of the main advantages of mixing collagen and CNF and using riboflavin/UVA crosslinking.

A CNF molecule cross-links with a collagen molecule in its vicinity, also called inter-fibril-molecular, this is generally done via short-range small molecule carbodiimide crosslinkers and larger size crosslinker, riboflavin in the presence of UVA. The larger spacing between CNF molecules allows riboflavin to penetrate in and creates crosslinks.

With UVA light exposure, or near UV, the intermediate hydrogel material is exposed to light having a wavelength of 315-400 nm. The solution is allowed to react in UVA for at least 1 min, or at least 5 min, or at least 10 min, such as 1-60 min, 5-60 min, 10-60 min or 15-60 min.

The solution in which the collagen molecules, the CNFs, the short-range small molecule carbodiimide crosslinkers and the riboflavin are mixed may be water or a buffer such as PBS. The collagen molecules may be dissolved in one solution and the CNFs in another solution, which then are mixed and thereafter the short-range carbodiimide crosslinkers and the riboflavin are added. Alternatively, the collagen molecules and the CNFs may be dissolved in the same solution.

The concentration of collagen in the formed mixture is 0.1-30 wt.%, or 0.5-30 wt.%, or 1-30 wt.%, or 5-30 wt.%, or 10-30 wt.%, or 15-30 wt.% or 20-30 wt.%, or 25-30 wt.%, or 0.1-25 wt.%, or 0.1-20 wt.%, or 0.1-15 wt.%, or 0.1-10 wt.%, 0.1-5 wt.%, or 0.1-1 wt.%, or 1-5 wt.%, or 5-10 wt.%, or 10-20 wt.%, or 20-25 wt.%.

The concentration of cellulose nanofiber in the solution is between 0.1 and 30 wt.%. At the higher concentrations of cellulose nanofibers, the hydrogel material might not be transparent or fully transparent (*i.e.,* having a transmission of at least 80%). Depending on application area, different degrees of transparency might be needed. If used for example in the eye, a transmission of 80% or more would be desirable, and a content of cellulose nanofibers of about 10 wt.% or lower would give the desired transparency.

The concentration of the carbodiimide crosslinking-agent in the mixture is 1-30 wt.%, or 5-30 wt.%, or 10-30 wt.%, or 15-30 wt.%, or 20-30 wt.%, or 25-30 wt.%, or 1-25 wt.%, or 1-20 wt.%, or 1-15 wt.%, or 1-10 wt.%, or 1-5 wt.%, or 5-10 wt.%, or 10-20 wt.%.

The concentration of riboflavin in the mixture is 0.1 to 10 wt.%, 0.5 to 10 wt.%, 1 to 10 wt.%, 2 to 10 wt.%, 3 to 10 wt.%, 4 to 10 wt.%, 5 to 10 wt.%, 6 to 10 wt.%, 7 to 10 wt.%, 8 to 10 wt.%, 9 to 10 wt.%, 2 to 8 wt.%, 2 to 6 wt.%, 2 to 4 wt.%, 0.1 to 1.0 wt.%, or 0.2 to 1.0 wt.%, or 0.3 to 1.0 wt.%, or 0.4 to 1.0 wt.%, or 0.5 to 1.0 wt.%, or 0.6 to 1.0 wt.%, or 0.7 to 1.0 wt.%, or 0.8 to 1.0 wt.%, or 0.9 to 1.0 wt.%, or 0.1 to 0.9 wt.%, or 0.1 to 0.8 wt.%, or 0.1 to 0.7 wt.%, or 0.1 to 0.6 wt.%, or 0.1 to 0.5 wt.%, or 0.1 to 0.4 wt.%, or 0.1 to 0.3 wt.%, 0.1 to 0.2 wt.%, or 0.2 to 0.4 wt.%, or 0.4 to 0.6 wt.%, 0.6 to 0.8 wt.%.

The concentration of cellulose nanofibers in the solution is 0.1-30 wt.%, or 0.5-30 wt.%, or 1-30 wt.%, or 5-30 wt.%, or 10-30 wt.%, or 15-30 wt.% or 20-30 wt.%, or 25-30 wt.%, or 0.1-25 wt.%, or 0.1-20 wt.%, or 0.1-15 wt.%, or 0.1-10 wt.%, or 1-5 wt.%, or 5-10 wt.%, or 0.2-10 wt.%, or 0.5-10 wt.%, or 0.8-10 wt.%, or 1-10 wt.%, or 2-10 wt.%, or 3-10 wt.%, or 4-10 wt.%, or 5-10 wt.%, or 6-10 wt.%, or 7-10 wt.%, or 8-10 wt.%, or 9-10 wt.%, or 0.1-9 wt.%, or 0.1-8 wt.%, or 0.1-7 wt.%, or 0.1-6 wt.%, or 0.1-5 wt.%, or 0.1-4 wt.%, or 0.1-3 wt.%, or 0.1-2 wt.%, or 0.1-1 wt.%, or 0.1-0.5 wt.%, or 0.5-5 wt.%, or 5-10 wt.%, or 2-8 wt.%.

A ratio of collagen to cellulose nanofibers in the solution may be 95:5 to 99.9:0.1.

The ratio may be 95:5 to 99.9:0.1, or 96:4-99.9:0.1, or 97:3-99.9:0.1, or 98:2-99.9:0.1, or 99:1-99.9:0.1, or 99.5:0.5-99.9:0.1, or 95:5-99.5:0.5, or 95:5-99:1.0, or 95:5-98:2.0, or 95:5-97:3.0, or 95:5-96:4.

The non-polymeric short-range carbodiimide crosslinking agent may be selected from a group consisting of ethyl-N'-(3-dimethylaminopropyl) carbodiimide hydrochloride (EDC), 1 -[3-(dimethylamino)propyl]-3-ethylcarbodiimide methiodide (EDCM), dicyclohexyl- carbodiimide (DCC), N-hydroxy-succinimide (NHS) and combinations thereof.

The collagen may be selected from a group consisting of Type I collagen, Type II collagen, Type III collagen, Type IV collagen, Type V collagen, Type VI collagen, denatured collagen from animal sources, and/or human recombinant collagens.

Twenty-nine types of collagens have been identified, but types I, II and III are the most abundant and make up the majority of the extracellular matrix macromolecules and have several roles. For instance, over 90% of the collagen in the human body is type I. All fibril-forming collagens (e.g., types I, II, III, V, XI, etc.) may be used in the hydrogel material. This is because many of the crosslinking sites that are needed for formation of crosslink networks are common among these collagens. In addition, these collagens are protein complexes whose basic units consist of the same triple helices (tropocollagen) in which three polypeptide chains are wound around each other like a piece of rope.

The collagen can be derived from different sources such as animals' tissue (e.g., pig skin, jelly fish, fish scale, human, etc.) or synthetically produced collagens such as human recombinant collagen.

Collagen molecules can self-assemble into micro-fibrils and then fibrils. For tissue engineering applications, one collagen type or a combination of various types can be used depending on the target tissue or organ that need to be replaced or repaired. For example, collagen type I is abundant in the human cornea, skin, tendon, and bone while collagen type II is abundant in cartilage and type III is abundant in veins Collagen type III is more abundant in skin, lung, cornea, and the vascular system, frequently in association with type I collagen.

The cellulose nanofibers may be derived from biomass, plants, and/or bacteria.

Nanocellulose can be sourced from biomass, plants, or bacteria. Nanocellulose derived from pulp and medical grade nanocellulose derived from Ciona Intestinalis may be used.

Ciona tunicates are the only animals known to produce cellulose. It is believed that they acquired this ability through lateral gene transfer from cyanobacteria. Ciona is also an environmentally friendly source of nanocellulose as instead of cutting trees, it is produced from Ciona species which are aggressively growing in the ocean and costal sea areas resulting in depletion of nutrients for other sea creators and plants as well as blockage of the water inlet to factories that relay on sea water for their processing and causing trouble for boat and ship loading docks.

The cellulose nanofibers (CNFs) are composed of nano-sized cellulose fibrils with a high aspect ratio (length to width ratio). CNF from Ciona has an aspect ratio of about 396. Typical fibril widths are 5-20 nanometers with a wide range of lengths, typically several micrometers.

Ciona nanocellulose is longer and wider and thus stronger than CNFs from e.g., wood. For example, Ciona CNF has a length of 1000-3000 nm which is more than 10-folds longer than woody CNF at 100-150 nm. Ciona CNF has a diameter range of 15-30 nm which is higher than 4-10 nm for woody CNFs.

Ciona tunics are rich in cellulose (44%) and do not contain lignin, facilitating cellulose extraction and purification up to 99.5% especially for medical application. The source of Ciona CNF used is of a medical grade quality, which is a must for applications in tissue engineering while there is no woody medical grade CNF to our knowledge.

A molar ratio of the non-polymeric short-range carbodiimide crosslinking agent to riboflavin in the solution may be from 10:1-500:1.

The molar ratio of the non-polymeric short-range carbodiimide crosslinking agent to riboflavin may be from 10:1-500:1, or 50:1-500:1, or 100:1-500:1, or 200:1-500:1, or 300:1-500:1, or 400:1-500:1, or 10:1-400:1, or 10:1-300:1, or 10:1-200:1, or 10:1-100:1, or 100:1-300:1.

The pH of the solution may be 3-6.

pH plays a key role in the crosslinking efficiency and therefore is a tool to control degradation gradient within the hydrogel material. The pH of the solution may be 3-6, or 4-6, or 5-6, or 3-4, or 3-5, or 4-5.

The method may further comprise a step of rinsing the formed intermediate hydrogel material before exposing the intermediate hydrogel material to ultraviolet A light.

The intermediate hydrogel material may be rinsed with water or a buffer such as PBS.

According to a second aspect, there is provided an implantable hydrogel material as claimed comprising collagen in a concentration of 0.1-30 wt.%, wherein the collagen molecules are crosslinked.

The collagen molecules are both intra- and inter-molecularly crosslinked. Collagen molecules are both chemically and physically crosslinked.

The degree of crosslinking may be as high as 100%, or 80-100% crosslinked.

The content of collagen in the hydrogel material is 0.1 -30 wt.%, or 0.5-30 wt.%, or 1-30 wt.%, or 5-30 wt.%, or 10-30 wt.%, or 15-30 wt.% or 20-30 wt.%, or 25-30 wt.%, or 0.1-25 wt.%, or 0.1-20 wt.%, or 0.1-15 wt.%, or 0.1-10 wt.%, 0.1-5 wt.%, or 0.1-1 wt.%, or 1-5 wt.%, or 5-10 wt.%, or 10-20 wt.%, or 20-25 wt.%.

Such a hydrogel material is highly transparent, biocompatible, and mechanically strong and can tolerate surgical manipulations, sutures, and remain stable and intact when implanted *in vivo.*

The implantable hydrogel material further comprises cellulose nanofibers in a concentration of 0.1-30 wt.%, wherein the cellulose nanofibers are crosslinked, and wherein there are crosslinks between the cellulose nanofibers and the collagen molecules.

There are both intra- and inter-fibrillary crosslinks and inter fibrillary-molecular crosslinks. The collagen molecules and the cellulose nanofibers are chemically and physically crosslinked. The degree of crosslinking may be as high as 100%, or 80-100% crosslinked.

The content of cellulose nanofibers in the formed hydrogel is 0.1-30 wt.%, or 0.5-30 wt.%, or 1-30 wt.%, or 5-30 wt.%, or 10-30 wt.%, or 15-30 wt.% or 20-30 wt.%, or 25-30 wt.%, or 0.1-25 wt.%, or 0.1-20 wt.%, or 0.1-15 wt.%, or 0.1-10 wt.%, or 1-5 wt.%, or 5-10 wt.%, or 0.1-10 wt.%, or 0.2-10 wt.%, or 0.5-10 wt.%, or 0.8-10 wt.%, or 1-10 wt.%, or 2-10 wt.%, or 3-10 wt.%, or 4-10 wt.%, or 5-10 wt.%, or 6-10 wt.%, or 7-10 wt.%, or 8-10 wt.%, or 9-10 wt.%, or 0.1-9 wt.%, or 0.1-8 wt.%, or 0.1-7 wt.%, or 0.1-6 wt.%, or 0.1-5 wt.%, or 0.1-4 wt.%, or 0.1-3 wt.%, or 0.1-2 wt.%, or 0.1-1 wt.%, or 0.1-1 wt.%, or 0.5-5 wt.%, or 5-10 wt.%, or 2-8 wt.%.

A ratio of collagen molecules to cellulose nanofibers in the hydrogel material is 95:5 to 99.9:0.1, or 96:4-99.9:0.1, or 97:3-99.9:0.1, or 98:2-99.9:0.1, or 99:1-99.9:0.1, or 99.5:0.5-99.9:0.1, or 95:5-99.5:0.5, or 95:5-99:1.0, or 95:5-98:2.0, or 95:5-97:3.0, or 95:5-96:4.

The hydrogel material may exhibit a light transmission of at least 80%.

The hydrogel material may be essentially transparent having a light transmission of 80% or more. At higher concentrations of cellulose nanofibers than 10 wt.% in the hydrogel material, the light transmission through the hydrogel material is lower than 80% and, hence not of interest in ophthalmic applications, which require high transmission through the material.

The implantable hydrogel material may be loadable with cells, tissue factors, growth factors, bioactive agents and/or drugs.

The hydrogel material may be loaded with cells, tissue factors, growth factors, bioactive agents and drugs, which may be delivered to the area of implantation improving and increasing tissue regeneration.

According to a third aspect, there is provided the implantable hydrogel material described above for use in ophthalmic devices, skin replacement, cardiac wall repairs, or cardiac patch applications.

The ophthalmic device may be a corneal implant, a corneal lens, or a contact lens.

The corneal implant may be a full or partial corneal implant, a corneal inlay or a corneal onlay.

The hydrogel materials disclosed in the embodiments of the present invention can be bio-printed into implantable 3D architectures and morphologies for tissue engineering applications.

### BRIEF DESCRIPTION OF THE DRAWINGS

Fig. 1 shows tensile stress at maximum load of double crosslinked collagen material (Double-Xlinked), double crosslinked collagen-CNF material (CNF-double-Xlinked), and single crosslinked collagen material (Single Xlinked).
Fig. 2 shows tensile strain at maximum load of double crosslinked collagen material (Double-Xlinked), double crosslinked collagen-CNF material (CNF-double-Xlinked), and single crosslinked collagen material (Single Xlinked).
Fig. 3 shows energy at maximum load of double crosslinked collagen material (Double-Xlinked), double crosslinked collagen-CNF material (CNF-double-Xlinked), and single crosslinked collagen material (Single Xlinked).
Fig. 4 shows elastic modulus of double crosslinked collagen material (Double-Xlinked), double crosslinked collagen-CNF material (CNF-double-Xlinked), and single crosslinked collagen material (Single Xlinked).
Fig. 5 shows light transmission through double crosslinked collagen material (Double-Xlinked), double crosslinked collagen-CNF material (CNF-double-Xlinked), single crosslinked collagen material (Single Xlinked) and human cornea samples measured across the UV and visible light spectrum (200 - 700 nm) at room temperature,
Fig. 6A and 6B show microscopic images of human corneal epithelial cells grown on bioengineered scaffolds of double crosslinked collagen and double crosslinked collagen-cellulose nanofibers, respectively.
Figs 7A-7F shows the capability of the double crosslinked collagen-CNF for drug loading and slow drug release.
Figs 8a and 8b show optical coherence tomography (OCT) images of cross section and pachymetry maps of a patient's cornea before and after surgical insertion of a bioengineered corneal implant (double crosslinked collagen material (Double-Xlinked)).
Fig. 9 illustrates possible crosslinking scenarios/types of collagen and cellulose nanofibers using a non-polymeric short-range carbodiimide crosslinking agent and riboflavin plus UVA.

### DETAILED DESCRIPTION

Below is described an implantable collagen-based material and its production. The material is both physically and chemically stabilized, thereby forming a mechanically robust, elastic and biocompatible implantable material, which support and maintain tissue structure. The material is further transparent, cell friendly and bio-interactive, allowing seamless host-biomaterial integration that helps restore tissue functionality and avoid rejection of the material from the implant site.

An implantable collagen-based hydrogel material is disclosed below but not part of the invention, this material may be formed by mixing a collagen molecule solution, having a concentration of collagen molecules in water or buffer of 0.1 to 30 wt.%, with a non-polymeric short-range carbodiimide crosslinking agent and riboflavin. The formed mixture having a concentration of the non-polymeric short-range carbodiimide crosslinking-agent of 1-30 wt.%, and a concentration of riboflavin of 0.1 to 10 wt.%. The mixture is allowed to react, forming an intermediate hydrogel material. The step of allowing the mixture to react, forming an intermediate hydrogel material may take place in a mould shaping the hydrogel material into its wanted shape, such as a lens or cornea etc. The formed intermediate hydrogel material may optionally be rinsed with water of a buffer such as PBS. The intermediate hydrogel material is exposed to ultraviolet A light, thereby forming an implantable hydrogel material. The formed material is a double-crosslinked collagen material, physically and chemically stabilized.

The hydrogel material may be reinforced by adding an amount of cellulose nanofibers in the solution during the production of the hydrogel material. A concentration of cellulose nanofibers added in the solution may be 0.1 to 30 wt.%. Such a hydrogen reinforced material has an increased mechanical robustness as compared to the hydrogel material without cellulose nanofibers, while elasticity, biocompatibility and transparency are maintained.

Depending on the amount of nanocellulose fibers in the mixture, the hydrogel material formed may be a pure double crosslinked (*i.e.,* crosslinked by short range carbodiimide crosslinker and crosslinked using riboflavin and UVA) collagen material or a collagen material re-inforced with an amount of nanocellulose.

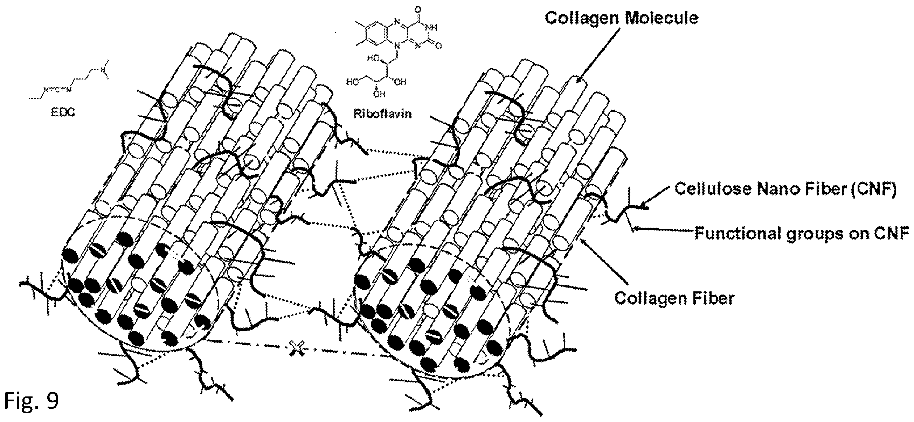

Double chemical-photochemical crosslinking of collagen and collagen reinforced with cellulose nanofibers (CNF) is shown in the schematics in Fig. 9. There are six possible crosslinking scenarios/types. These scenarios/types vary depending on the molecules or fibers that are being linked together and the type of crosslinkers.
**Type 1:** A collagen molecule only cross-links with itself, also called intra-molecular cross-linking, this is generally done via short-range carbodiimide crosslinkers, such as EDC/NHS. Crosslinkers such as riboflavin are not expected to result in such crosslinks due to their larger molecular size than the carbodiimide and their bulky cyclic groups (e.g., isoalloxazine) that limit their penetration depth into the molecules.
**Type 2:** A CNF molecule (nanofiber) only cross-links with itself, which is also called intra-molecular crosslinking. This is generally done via short-range carbodiimide crosslinkers, such as EDC/NHS.
**Type 3:** A collagen molecule cross-links with another collagen molecule in its vicinity, also called inter-molecular crosslinking. This is generally done via short-range carbodiimide crosslinkers, such as EDC/NHS, and larger size crosslinkers such as riboflavin in the presence of UVA. The larger spacing between collagen molecules allows riboflavin to penetrate in and creates crosslinks.
**Type 4:** A CNF molecule (nanofiber) cross-links with another CNF molecule (nanofiber), also called inter-molecular or inter-fibrillar crosslinking. This is generally done via short-range carbodiimide crosslinkers, such as EDC/NHS, when the CNFs are in each other's vicinity and/or by larger size crosslinkers, such as riboflavin, in the presence of UVA when CNFs are more distanced. The larger spacing between CNF molecules allows riboflavin to penetrate in and creates crosslinks. This type of crosslinks can also occur between two CNF fibers that are attached to two different collagen fibers creating a bridge between two collagen fibers.
**Type 5:** A CNF molecule cross-links with a collagen molecule in its vicinity, also called inter-fibril-molecular crosslinking. This is generally done via short-range carbodiimide crosslinkers, such as EDC/NHS, and larger size crosslinkers such as riboflavin in the presence of UVA. The larger spacing between CNF molecules allows riboflavin to penetrate in and creates crosslinks. **Type 6:** A collagen fiber cross-links with another collagen fiber in its vicinity, also called inter-fibrillar crosslinking. This type of crosslink may be done via long-range large molecule crosslinkers such as PEG-dialdehyde. Short-range carbodiimide crosslinkers such as EDC/NHS cannot produce such crosslinks because of the large spacing between the collagen fibers. However, larger size crosslinkers such as riboflavin in the presence of CNF and UVA can result in crosslinks between two collagen fibers.

Double-crosslinked collagen, double crosslinked collagen-CNF and single crosslinked collagen (using EDC/NHS) formulations are associated with certain types of crosslinks described above. For example, it is believed that the crosslinks in single crosslinked collagen hydrogel materials are of type 1 and 3, in double crosslinked collagen hydrogel material crosslinks of type 1, 3, and 6 are present, while the ones in double crosslinked collagen-CNF are of all types from type 1 to type 6.

The implantable hydrogel material described above may be used in ophthalmic devices, skin replacement, cardiac wall repairs, or cardiac patch applications. The ophthalmic device may be a corneal implant, a corneal lens, or a contact lens.

In the experimental section below is a described non-limiting examples of production of such implantable hydrogel materials, and different tests thereof.

### EXPERIMENTAL

### Collagen

For this work purified medical-grade type I porcine collagen from porcine skin was used.

### Cellulose nano fibers

In the experiments described here, nanocellulose derived from pulp and medical grade nanocellulose derived from *Ciona Intestinalis* were used.

### Buffer

MES buffer (2-(N-morpholino) ethanesulfonic acid) at a concentration range of 0.5 to 1 Molar was used for fabrication of the hydrogel material/scaffolds. Sterile phosphate buffered saline (PBS) was used as storage buffer.

### Crosslinkers

1-Ethyl-3-(3-dimethylaminopropyl)carbodiimide (EDC), 1-[3-(Dimethylamino) propyl]-3-ethylcarbodiimide methiodide (EDCM), N-hydroxysuccinimide (NHS), and riboflavin (known as vitamin B₂) 7,8-dimethyl-10-(D-ribo-2,3,4,5-tetrahydroxypentyl)isoalloxazine and 7,8-dimethyl-10-ribitylisoalloxazine were used as crosslinking agents

### Material 1 - double-crosslinked collagen (not according to the invention)

Medical grade purified freeze-dried type I collagen was dissolved in water or PBS at 20 wt.% solution concentration. The collagen solution was then mixed with crosslinkers 1-[3-(Dimethylamino) propyl]-3-ethylcarbodiimide methiodide (EDCM), 1-Ethyl-3-(3-dimethylaminopropyl)carbodiimide (EDC), N-hydroxysuccinimide (NHS) at 20%, and riboflavin (vitamin B2) at 0.5% concentration. The solution was mixed thoroughly, dispensed into custom moulds, clamped and then cured. Removal from moulds was achieved by immersion in PBS for one hour at room temperature. Scaffolds were immersed in riboflavin and then exposed to UVA. Finally, scaffolds were rinsed with sterile PBS to extract any reaction residues.

### Material 2 - double crosslinked collagen-CNF

Medical grade purified freeze-dried type I collagen was dissolved in water or PBS at 20% solution concentration. Nanocellulose solution at 2.5% was added to the collagen solution and then mixed with crosslinkers 1-[3-(Dimethylamino) propyl]-3-ethylcarbodiimide methiodide (EDCM),1-ethyl-3-(3-dimethylaminopropyl)carbodiimide (EDC), N-hydroxysuccinimide (NHS) at 20%, and riboflavin (vitamin B2) at 0.5% concentration. The solution was mixed thoroughly, dispensed into custom moulds, clamped and then cured. Removal from moulds was achieved by immersion in PBS for one hour at room temperature. Scaffolds were immersed in riboflavin and then exposed to UVA. Finally, scaffolds were rinsed with sterile PBS to extract any reaction residues.

### Reference material - single crosslinked collagen

Medical grade purified freeze-dried type I collagen was dissolved in water or PBS at 20% solution concentration. The collagen solution was then mixed with crosslinker solutions of 1-[3-(Dimethylamino) propyl]-3-ethylcarbodiimide methiodide (EDCM), 1-ethyl-3-(3-dimethylaminopropyl)carbodiimide (EDC), and N-hydroxysuccinimide (NHS) at 20%. The solution was mixed thoroughly and then dispensed into custom moulds. Moulds were clamped and samples were cured. Removal from moulds was achieved by immersion in PBS for one hour at room temperature. Finally, scaffolds were rinsed with sterile PBS to extract any reaction residues.

### Mixing and molding

The solution was mixed thoroughly and dispensed into custom moulds with spacers of various thicknesses to delineate the final device thickness. Moulds were clamped and samples were cured at room temperature followed by curing at 37°C. Removal from moulds was achieved by immersion in PBS for one hour at room temperature. Finally, the moulded material was rinsed with sterile PBS to extract any reaction residues.

### 3D Bioprinting

The solution was mixed thoroughly with chemical crosslinkers and bio-printed into 3D custom shapes of various sizes to delineate the final device architecture followed by curing at 37°C. Moulds maybe used to further shape the implants if necessary.

### UVA

Following the chemical crosslinking, a photochemical crosslinking of samples was performed by immersion in riboflavin solution and exposure to ultraviolet A (UVA) light for 15-60 minutes at a wavelength of 365 nm and intensity of 1300µW/cm².

### RESULTS

### Selective Comparative Test Results (Mechanical, Transparency, and Cell Biocompatibility):

Double crosslinked collagen and double crosslinked collagen-CNF, prepared as discussed above, were tested and compared to single crosslinked collagen formulations for major physical and biological properties including mechanical properties, transparency (% light transmission), and cell biocompatibility.

### Mechanical strength, strain, energy at break, and elastic modulus

Tensile strength, elongation at break (elasticity), elastic modulus (stiffness), and energy at break (toughness) were measured with an Instron Automated Materials Testing System (Model 5943 Single Column Table Frame) equipped with BluHill software, a load cell of 50N capacity and pneumatic metal grips at a crosshead speed of 10 mm/min. Test specimens were made by molding and curing the samples into dumbbell-shaped Teflon molds followed by equilibration in PBS. Specimens were attached to the grips and tensile force was applied until the sample break point. Data were automatically recorded by the software, and 66 dumbbell-shaped samples were used for each mechanical test.

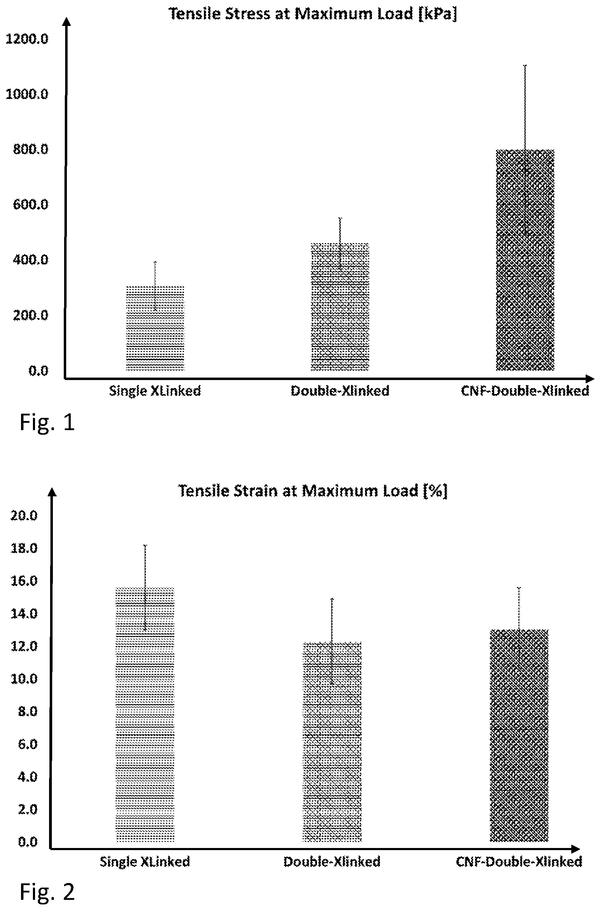

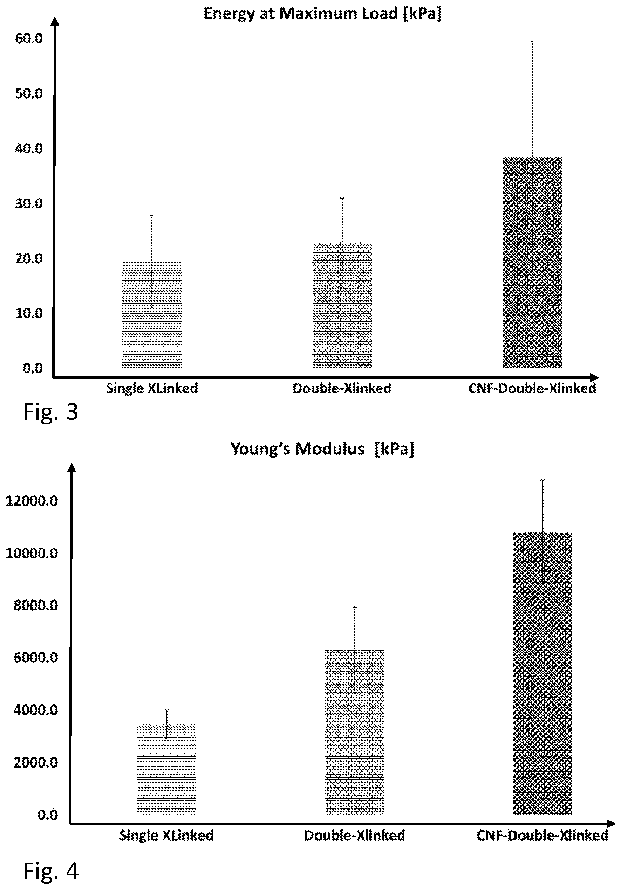

Double crosslinked collagen (Double Xlinked), and double crosslinked collagen-CNF (CNF-double-Xlinked), were tested and compared to single crosslinked collagen (Single Xlinked) formulations. In Fig. 1 is shown tensile stress, Fig. 2 shows tensile strain, Fig. 3 shows energy at maximum load, and Fig. 4 the Young's modulus.

The results suggest significant increases in stress (strength of the scaffold), energy at maximum load (robustness), and elastic modulus (stiffness) for the double crosslinked collagen and double crosslinked collagen-CNF compared to single crosslinked collagen, without sacrificing the strain (elasticity).

### Light transmission

Light transmission through the hydrogel material was measured across the UV and visible light spectrum (200 - 700 nm) at room temperature, using a High Performance USB4000 UV-Vis Spectrophotometer (Mettler Toledo, Stockholm). To enable direct comparison with light transmission through the native human cornea, the hydrogel material samples were 550µm thick. Samples were immersed in PBS during measurement, and light transmission was compared with published data from healthy human corneal tissue (Meek, K.M., et. al. Transparency, swelling and scarring in the corneal stroma. Eye (Lond) 17, 927-936 (2003). Measurements were made for three independent samples per spectrum.

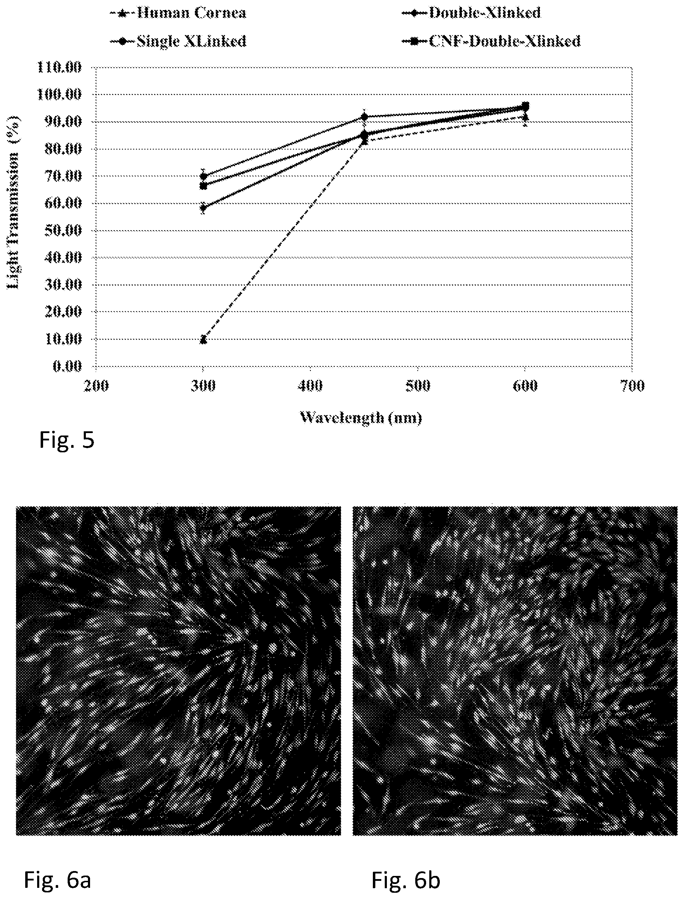

The light transmission results shown in Fig. 5, suggest that all three bioengineered formulations: double crosslinked collagen (Double-Xlinked), double crosslinked collagen-CNF (CNF-double-Xlinked), and single crosslinked collagen (Single Xlinked) have superior light transmission to the human cornea and that the significant increases in mechanical properties of the double-crosslinked and CNF-embedded double crosslinked scaffolds have not negatively impacted their transparency.

### Seeding with corneal epithelial cells

Culture of human corneal epithelial cells (HCE-2 50.B1 cell line, Lot No. 70015331, ATCC, USA) was established according to the manufacturer's instructions. Briefly, serum-free keratinocyte growth medium (1X, Gibco) was supplemented with L-Glutamine, 5 ng/ml epidermal growth factor (EGF) and 0.05 mg/ml bovine pituitary extract (BPE), 500 ng/ml hydrocortisone and 0.005 mg/ml insulin (Gibco). A T-75 cell culture flask was precoated with a mixture of 0.01 mg/ml fibronectin, 0.03 mg/mL bovine collagen type 1 and 0.01 mg/mL bovine serum albumin, and was incubated overnight at 37°C. The next day, the excess coating was aspirated, and the flask was allowed to stand for 15 minutes before seeding the cells. The HCE-2 cell vial was thawed, and cells were seeded at a density of 104 cells/cm² on the precoated flask. Cells were incubated at 37°C at 5% CO₂, and growth media was changed every other day.

Hydrogel material, double crosslinked collagen and double crosslinked collagen-cellulose nanofibers, (300µm thick) precut to 8 mm diameter were rinsed in PBS and equilibrated in the complete keratinocyte serum-free medium for 2 hours in a humidified cell culture incubator. The hydrogel samples were then laid down (concave side down) onto the bottom of a 48-well cell culture plate and allowed to adhere to the bottom of the plate for 2 hours in an incubator at 37°C. Three wells were used for each hydrogel sample, and three wells were used as controls (*i.e.,* no biomaterial attached to the bottom of the well).

Upon confluence of the seeded HCE-2 cells in the culture flask, the cells were trypsinized with Trypsin-EDTA, then trypsinization was stopped with a complete growth medium and cells were harvested, counted, and seeded into the six prepared wells. Cells were seeded at a density of 105 cells/well for the control and the two hydrogel materials and incubated for 1 hour prior to adding additional media to reach a final volume of 200 µl of growth media per well. Growth media was changed every other day, and cells were maintained in culture for 16 days. On day 16, the cells were washed and covered with fresh media. Cells were stained with NucBlue Live cell stain (Hoechst 33342, Thermo Fisher Scientific) according to the manufacturer's instructions. Images of stained cells were captured using a Leica DMi8 inverted live-cell microscope under ultraviolet light excitation (385nm) to detect live cells with fluorescent blue nuclear stain. Brightfield images were additionally obtained to observe cell morphology.

In Figs 6a and 6b are shown epithelial cells grown and confluent on the double crosslinked collagen and double crosslinked collagen-CNF hydrogel material, respectively, suggesting the scaffolds having full cell biocompatibility.

### Drug loading and release from the scaffolds

The capability of the double crosslinked collagen-CNF for drug loading and slow release was also tested. Three model drugs, e.g., dexamethasone, Nerve Growth factor (NGF), and Artemin were separately loaded into double crosslinked collagen-CNF bioengineered corneal implants and tested *in vitro* and *in vivo* in rabbit corneas for drug release and key physical properties of the corneas including transparency and mechanical properties. Dexamethasone is a steroid medicine that is used to relieve the redness, itching, and swelling caused by eye infections and other conditions or procedures (*e.g*.,, eye surgery). Dexamethasone eye drops are used to treat inflammation of the eyes caused by allergies and certain conditions, including damage caused by chemical and thermal burns as well as treating eye pain and inflammation after eye surgery.

A 1-3 mg/ml solution of dexamethasone was prepared. 30 microliters of the solution was then injected into the collagen solution before adding the crosslinkers as explained above. Similar protocols were used for NGF and Artemin. Upon crosslinking, the drugs will be covalently bound to collagen molecules making it possible for a long-term slow and continuous release of the drugs (up to 60 days) from the scaffolds.

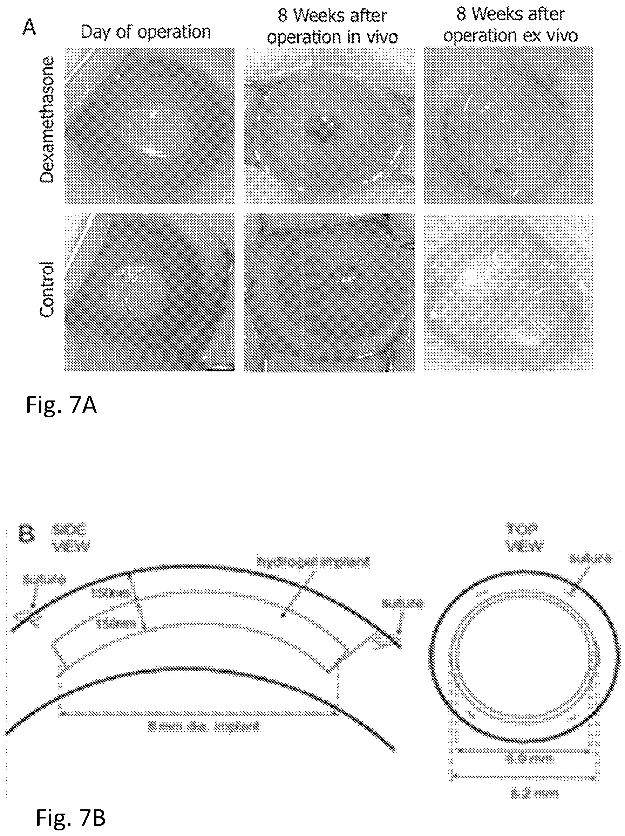

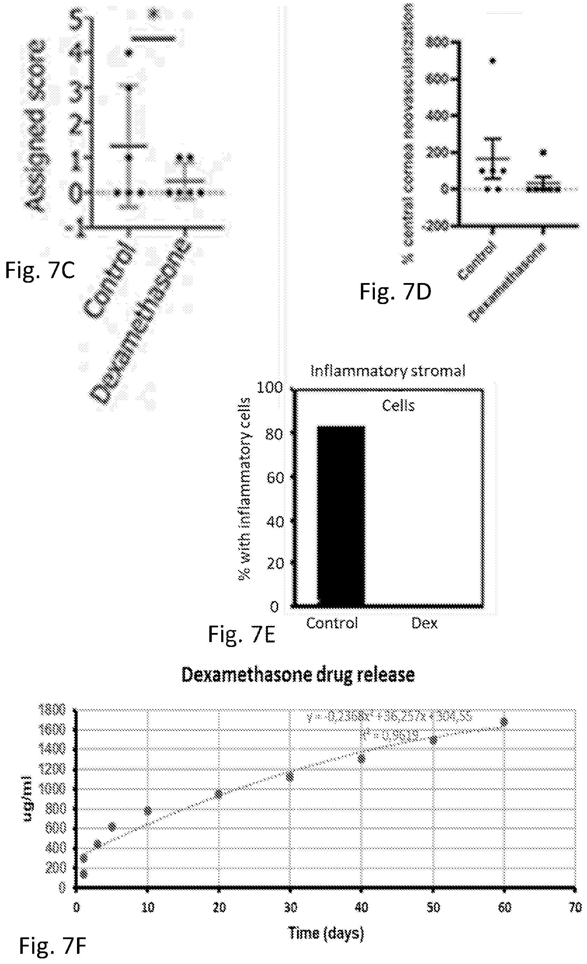

Fig. 7F shows the release profile of dexamethasone from implants over a 60-day period suggesting continuous slow release of dexamethasone. The dexamethasone-loaded implants along with drug-free implants (control) were then tested *in vivo* in rabbit models (Fig. 7A) by inserting them in a laser-created pocket (Fig. 7B). Thereafter, four sutures were placed circumferentially to stimulate a pathologic inflammatory response. The dexamethasone-loaded corneas performed significantly better than the controls with respect to haze prevention (Fig. 7C), reduced vascularization (Fig. 7D), and improved implant stability compared to drug-free implants (controls). The dexamethasone-loaded implants suppressed inflammation, by suppressing inflammatory cell infiltration (Fig. 7E) and expression of multiple cytokines in the cornea and aqueous humor fluid, as well as suppression of migration and tube formation by Human umbilical vein endothelial cells (HUVECs) *in vitro.*

### Proof-of-concept

No prior work to our knowledge has demonstrated a commercially and clinically viable, GMP-grade bioengineered scaffold from a sustainably sourced, cost-effective, widely available, and FDA-approved raw material, and no other technology has achieved manufacturability, packaging, sterility, or long shelf life that are ISO-compliant and independently third-party certified. Yet these often-overlooked aspects are critical for addressing the lack of donor tissue.

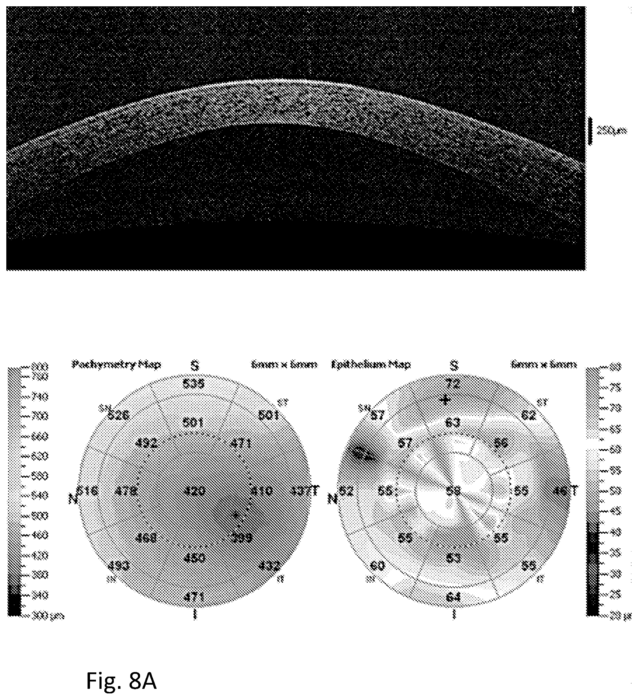

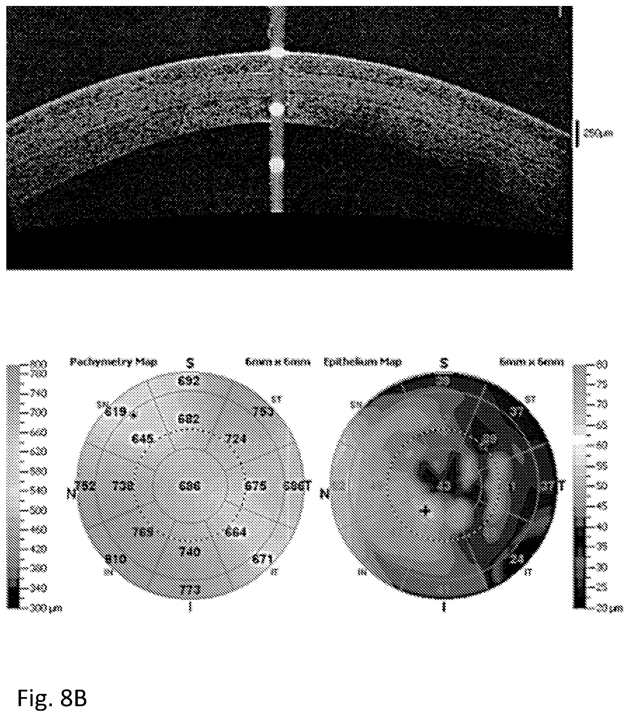

The optimum scaffold (double crosslinked collagen material (Double-Xlinked)) was tested in human corneas of 20 patients and data collected over 12 months post implantation. From a safety perspective, the scaffold did not result in thinning, loss of transparency, neovascularization, rejection, or other adverse event observed to varying degrees in most preclinical and clinical studies conducted by other groups. Figs 8A and 8B show the cross-section and topography of a patient cornea before (Fig. 8A) and after implantation of the selected scaffold (Fig. 8B). Regarding efficacy, no prior study has achieved full corneal transparency *in vivo* with significant corneal thickening (Fig. 8A) or with the significant visual acuity gains as reported here; at best only modest vision gains have been achieved in prior studies.

The scaffolds are mechanically more robust than earlier versions we previously developed, with superior toughness, elastic modulus, and stress tolerance translating into an ability to withstand surgical implantation, mechanical forces and enzymes *in vivo.* Our results suggest it may not be necessary for bioengineered tissue to match the mechanical properties of the native cornea to be therapeutically effective. Standard corneal transplantation with mechanically tough human donor tissue often results in scar tissue formation at host-to-implant interfaces, a phenomenon we did not observe with the mechanically softer scaffolds.

## Claims

1. Method of providing an implantable hydrogel material, the method comprising:
- providing a solution having a collagen concentration of 0.1 to 30 wt.% and a cellulose nanofiber concentration of 0.1 to 30 wt.%;
- adding to the solution a non-polymeric short-range carbodiimide crosslinking agent to a concentration of 1-30 wt.%,
- adding to the solution riboflavin to a concentration of 0.1 to 10 wt.%;
- mixing the solution;
- allowing the solution to react, forming an intermediate hydrogel material;
- exposing the intermediate hydrogel material to ultraviolet A light, thereby forming an implantable hydrogel material.

2. The method of claim 1, wherein a ratio of collagen to cellulose nanofibers in the mixture is 95:5 to 99.9:0.1.

3. The method of any of claims 1 to 2, wherein the non-polymeric short-range carbodiimide crosslinking agent is selected from a group consisting of ethyl-N'-(3-dimethylaminopropyl) carbodiimide hydrochloride (EDC), 1-[3-(dimethylamino)propyl]-3-ethylcarbodiimide methiodide (EDCM), dicyclohexyl- carbodiimide (DCC), N-hydroxy-succinimide (NHS) and combinations thereof.

4. The method of any of the preceding claims, wherein the collagen is selected from a group consisting of Type I collagen, Type II collagen, Type III collagen, Type IV collagen, Type V collagen, Type VI collagen, denatured collagen from animal sources, and/or human recombinant collagens.

5. The method of any of the preceding claims, wherein the cellulose nanofibers are derived from biomass, plants, and/or bacteria.

6. The method of any of the preceding claims, wherein a molar ratio of the non-polymeric short-range carbodiimide crosslinking agent to riboflavin in the solution is 10:1-500:1.

7. The method of any of the preceding claims, wherein pH of the solution is 3-6.

8. The method of any of claims 1-7, further comprising a step of rinsing the formed intermediate hydrogel material before exposing the intermediate hydrogel material to ultraviolet A light.

9. An implantable hydrogel material comprising collagen in a concentration of 0.1-30 wt.% and cellulose nanofibers in a concentration of 0.1-30 wt.%, wherein a ratio of collagen molecules to cellulose nanofibers in the hydrogel material is 95:5 to 99.9:0.1 and the degree of crosslinking of the hydrogel material is 80-100%, wherein collagen molecules are intra- and inter-molecularly crosslinked and chemically and physically crosslinked, wherein the cellulose nanofibers are intra- and inter-fibrillarly crosslinked and chemically and physically crosslinked, and wherein there are crosslinks between the cellulose nanofibers and the collagen molecules..

10. The implantable hydrogel material of claim 9, wherein the hydrogel material exhibits a light transmission of at least 80%, as measured across the UV and visible light spectrum (200 - 700 nm) at room temperature, using a UV-Vis spectrophotometer on a 550µm thick hydrogel material.

11. The implantable hydrogel material of any of claims 9-10, wherein the hydrogel material is loadable with cells, tissue factors, growth factors, bioactive agents and/or drugs.

12. The implantable hydrogel material of any of claims 9-11, for use in ophthalmic devices, skin replacement, cardiac wall repairs, or cardiac patch applications.

13. The implantable hydrogel material for use as in claim 12, wherein the ophthalmic device is a corneal implant, a corneal lens, or a contact lens.

## Patentansprüche

1. Verfahren zum Bereitstellen eines implantierbaren Hydrogelmaterials, wobei das Verfahren Folgendes umfasst:
- Bereitstellen einer Lösung, die eine Kollagenkonzentration von 0,1 bis 30 Gew.-% und eine Zellulose-Nanofaser-Konzentration von 0,1 bis 30 Gew.-% aufweist;
- Hinzufügen eines nicht-polymeren kurzreichweitigen Carbodiimid-Vernetzungsmittels zu der Lösung in einer Konzentration von 1-30 Gew.-%,
- Hinzufügen von Riboflavin zu der Lösung in einer Konzentration von 0,1 bis 10 Gew.-%;
- Mischen der Lösung;
- Zulassen der Reaktion der Lösung, wodurch ein intermediäres Hydrogelmaterial gebildet wird;
- Aussetzen des intermediären Hydrogelmaterials gegenüber ultraviolettem A-Licht, wodurch ein implantierbares Hydrogelmaterial gebildet wird.

2. Verfahren nach Anspruch 1, wobei ein Verhältnis von Kollagen zu Zellulose-Nanofasern in dem Gemisch 95:5 bis 99,9:0,1 beträgt.

3. Verfahren nach einem der Ansprüche 1 bis 2, wobei das nichtpolymere kurzreichweitige Carbodiimid-Vernetzungsmittel ausgewählt ist aus einer Gruppe, die besteht aus Ethyl-N'-(3-dimethylaminopropyl)carbodiimidhydrochlorid (EDC), 1-[3-(Dimethylamino)propyl]-3-ethylcarbodiimidmethiodid (EDCM), Dicyclohexylcarbodiimid (DCC), N-Hydroxysuccinimid (NHS) und Kombinationen davon.

4. Verfahren nach einem der vorhergehenden Ansprüche, wobei das Kollagen ausgewählt ist aus einer Gruppe, die besteht aus Typ I Kollagen, Typ II Kollagen, Typ III Kollagen, Typ IV Kollagen, Typ V Kollagen, Typ VI Kollagen, denaturiertem Kollagen aus tierischen Quellen und/oder rekombinantem menschlichem Kollagen.

5. Verfahren nach einem der vorhergehenden Ansprüche, wobei die Zellulose-Nanofasern gewonnen werden aus Biomasse, Pflanzen und/oder Bakterien.

6. Verfahren nach einem der vorhergehenden Ansprüche, wobei ein molares Verhältnis des nicht-polymeren kurzreichweitigen Carbodiimid-Vernetzungsmittels zu Riboflavin in der Lösung 10:1-500:1 beträgt.

7. Verfahren nach einem der vorhergehenden Ansprüche, wobei der pH der Lösung 3-6 beträgt.

8. Verfahren nach einem der Ansprüche 1-7, ferner umfassend einen Schritt des Spülens des gebildeten intermediären Hydrogelmaterials vor dem Aussetzen des intermediären Hydrogelmaterials gegenüber ultraviolettem A-Licht.

9. Implantierbares Hydrogelmaterial, umfassend Kollagen in einer Konzentration von 0,1-30 Gew.-% und Zellulose-Nanofasern in einer Konzentration von 0,1-30 Gew.-%, wobei ein Verhältnis der Kollagenmoleküle zu Zellulose-Nanofasern in dem Hydrogelmaterial 95:5 bis 99,9:0,1 beträgt und der Grad der Vernetzung des Hydrogelmaterials 80-100 % beträgt, wobei die Kollagenmoleküle intra- und intermolekular vernetzt und chemisch und physikalisch vernetzt sind, wobei die Zellulose-Nanofasern intra- und interfibrillär vernetzt und chemisch und physikalisch vernetzt sind, und wobei es Vernetzungen gibt zwischen den Zellulose-Nanofasern und den Kollagenmolekülen..

10. Implantierbares Hydrogelmaterial nach Anspruch 9, wobei das Hydrogelmaterial eine Lichtdurchlässigkeit von mindestens 80 % aufweist, gemessen über das UV- und sichtbare Lichtspektrum (200 - 700 nm) bei Raumtemperatur unter Verwendung eines UV-Vis-Spektralphotometers an einem 550 µm dicken Hydrogelmaterial.

11. Implantierbares Hydrogelmaterial nach einem der Ansprüche 9-10, wobei das Hydrogelmaterial mit Zellen, Gewebefaktoren, Wachstumsfaktoren, bioaktiven Wirkstoffen und/oder Medikamenten beladen werden kann.

12. Implantierbares Hydrogelmaterial nach einem der Ansprüche 9-11 zur Verwendung in ophthalmologischen Geräten, Hautersatz, Herzwandreparaturen oder Herzpflasteranwendungen.

13. Implantierbares Hydrogelmaterial zur Verwendung nach Anspruch 12, wobei das ophthalmologische Gerät ein Hornhautimplantat, eine Hornhautlinse oder eine Kontaktlinse ist.

## Revendications

1. Procédé de fourniture d'un matériau hydrogel implantable, le procédé comprenant :
- la fourniture d'une solution ayant une concentration en collagène de 0,1 à 30 % en poids et une concentration en nanofibres de cellulose de 0,1 à 30 % en poids ;
- l'ajout à la solution d'un agent de réticulation carbodiimide non polymérique à courte portée à une concentration de 1 à 30 % en poids,
- l'ajout à la solution de la riboflavine à une concentration de 0,1 à 10 % en poids ;
- le mélange de la solution ;
- la permission à la solution de réagir, formant un matériau hydrogel intermédiaire ;
- l'exposition du matériau hydrogel intermédiaire à la lumière ultraviolette A, formant ainsi un matériau hydrogel implantable.

2. Procédé selon la revendication 1, dans lequel le rapport entre les nanofibres de collagène et de cellulose dans le mélange est de 95 : 5 à 99,9 : 0,1.

3. Procédé selon l'une quelconque des revendications 1 à 2, dans lequel l'agent de réticulation carbodiimide à courte portée non polymérique est choisi parmi un groupe constitué de chlorhydrate d'éthyl-N'-(3-diméthylaminopropyl)carbodiimide (EDC), de méthiodure de 1-[3-(diméthylaminopropyl]-3-éthylcarbodiimide (EDCM), de dicyclohexylcarbodiimide (DCC), de N-hydroxysuccinimide (NHS) et de combinaisons de ceux-ci.

4. Procédé selon l'une quelconque des revendications précédentes, dans lequel le collagène est choisi parmi un groupe constitué de collagène de type I, de collagène de type II, de collagène de type III, de collagène de type IV, de collagène de type V, de collagène de type VI, de collagène dénaturé d'origine animale et/ou de collagènes recombinants humains.

5. Procédé selon l'une quelconque des revendications précédentes, dans lequel les nanofibres de cellulose sont dérivées de biomasse, de plantes et/ou de bactéries.

6. Procédé selon l'une quelconque des revendications précédentes, dans lequel le rapport molaire de l'agent de réticulation carbodiimide à courte portée non polymérique à la riboflavine dans la solution est de 10 : 1 à 500 : 1.

7. Procédé selon l'une quelconque des revendications précédentes, dans lequel le pH de la solution est de 3 à 6.

8. Procédé selon l'une quelconque des revendications 1 à 7, comprenant en outre une étape de rinçage du matériau hydrogel intermédiaire formé avant d'exposer le matériau hydrogel intermédiaire à la lumière ultraviolette A.

9. Matériau hydrogel implantable comprenant du collagène à une concentration de 0,1 à 30 % en poids et des nanofibres de cellulose à une concentration de 0,1 à 30 % en poids, dans lequel le rapport des molécules de collagène par rapport aux nanofibres de cellulose dans le matériau hydrogel est de 95 : 5 à 99,9 : 0,1 et le degré de réticulation du matériau hydrogel est de 80 à 100 %, dans lequel les molécules de collagène sont réticulées de manière intra- et intermoléculaire et chimiquement et physiquement, dans lequel les nanofibres de cellulose sont réticulées de manière intra- et interfibrillaire et chimiquement et physiquement, et dans lequel il existe des réticulations entre les nanofibres de cellulose et les molécules de collagène.

10. Matériau hydrogel implantable selon la revendication 9, dans lequel le matériau hydrogel présente une transmission de lumière d'au moins 80 %, mesurée sur le spectre de la lumière UV et visible (200 à 700 nm) à température ambiante, à l'aide d'un spectrophotomètre UV-Vis sur un matériau hydrogel de 550 µm d'épaisseur.

11. Matériau hydrogel implantable selon l'une quelconque des revendications 9 à 10, dans lequel le matériau hydrogel est chargeable avec des cellules, des facteurs tissulaires, des facteurs de croissance, des agents bioactifs et/ou des médicaments.

12. Matériau hydrogel implantable selon l'une quelconque des revendications 9 à 11, destiné à être utilisé dans des dispositifs ophtalmiques, le remplacement de la peau, les réparations de la paroi cardiaque ou les applications de patchs cardiaques.

13. Matériau hydrogel implantable destiné à être utilisé selon la revendication 12, dans lequel le dispositif ophtalmique est un implant cornéen, une lentille cornéenne ou une lentille de contact.
